# EUROPÄISCHE PATENTSCHRIFT

(11) **EP 0 468 518 B1**
(45) Veröffentlichungstag und Bekanntmachung des Hinweises auf die Patenterteilung: **02.10.1996**
(21) Anmeldenummer: 91112598.7
(22) Anmeldetag: 26.07.1991
(51) Int. Cl.: A61L 25/00, A61K 6/083

(54) **Verwendung von Glasionomerzement für gesteuerte Gewebsregenerationen**
Use of glass ionomer cement for guided tissue regeneration
Utilisation de ciment ionomère-verre pour la régénération assisteé de tissu

(30) Priorität: 26.07.1990 DE 4023744
(43) Veröffentlichungstag der Anmeldung: 29.01.1992
(73) Patentinhaber: THERA Patent GmbH & Co. KG Gesellschaft für industrielle Schutzrechte, D-82229 Seefeld (DE)
(72) Erfinder: Ney, Thomas, Dr., W-7400 Tübingen 1 (DE); Zöllner, Werner, Dr., W-8031 Wörthsee/Steinebach (DE)
(74) Vertreter: Abitz, Walter, Dr.-Ing.

(56) Entgegenhaltungen:
- EP-A- 0 331 071
- FR-A- 2 370 468
- FR-A- 2 640 503

## Beschreibung

Durch parodontale Infektionen kommt es im Kieferbereich zum Abbau von Knochensubstanz, wodurch sich eine fortschreitende Lockerung und schliesslich der Verlust der befallenen Zähne ergibt. Die intermediär auftretenden Stadien können durch freiliegende Zahnhälse und freiliegende Bifurkationen (Bereich der Wurzelverzweigung der Seitenzähne) zu schmerzhaften Zuständen führen. Auch nach erfolgreicher Bekämpfung der Infektion ist ein Festwachsen der Zähne meist nicht mehr möglich, da der Knochendefekt nur durch Bindegewebe ausgefüllt wird. Dies ist die Folge daraus, dass das Bindegewebe beim konkurrierenden Ausfüllen des Knochendefekts wesentlich schneller wächst als die Knochensubstanz selbst. Eine knöcherne Regeneration (sogenanntes "Reattachment") findet also nur selten statt.

Bereits vor einiger Zeit wurde in der medizinischen Fachwelt das Konzept der gesteuerten Gewebsregeneration (Guided tissue regeneration, GTR) eingeführt. Dabei wird der Knochendefekt künstlich abgeschirmt und man lässt dann den durch die Abschirmung gebildeten Hohlraum einfach mit Blut ausfüllen. Die Abschirmung soll das Einwachsen des Bindegewebes verhindern, so dass eine knöcherne Regeneration in dem gebildeten Hohlraum stattfinden kann. Nach Entfernen der Abschirmung ist eine Wiederbefestigung des Zahnes somit erreichbar.

In der EP-B 0 171 173 werden als Abschirmmaterialien semipermeable Membranen aus Polytetrafluorethylen (PTFE) vorgeschlagen. Auch resorbierbare Gewebe zur Herstellung dieser Abschirmung sind in der Literatur beschrieben. In einem Vortrag von Dr. Carla Noppe anlässlich der Jahrestagung 1989 der Deutschen Gesellschaft für Parodontologie am 29. und 30. September 1989 in Aachen wurde über den praktischen Einsatz der semipermeablen PTFE-Membranen berichtet. Dabei wurde darauf hingewiesen, dass diese Membranen über eine offene Mikrostruktur mit ca. 10 µm Porengrösse verfügen, in welche gingivale Fibroblasten einwandern und ein Epitheltiefenwachstum zwischen Filter und Gingiva verhindern sollen. Bei den Versuchen wurden 40 Membranen verschiedener Konfiguration 4 bis 6 Wochen implantiert. Es zeigte sich bei der Auswertung, dass bei 39 Operationsgebieten ein Epitheltiefenwachstum von 2 bis 4 mm unter der Membran auftrat, und dass grosse Teile der Membranen bakteriell besiedelt waren. Obgleich somit durch die Verwendung derartiger Membranen und Geweben ein gewisser Erfolg erzielt werden kann, ist deren klinische Anwendung jedoch überaus schwierig. Das flexible Gewebe ist nur mühsam in situ zu befestigen, und darüber hinaus erfordert die geringe Biokompatibilität der PTFE-Membrane ein Entfernen aus dem Situs bereits nach wenigen Wochen. Ein Epitheltiefenwachstum lässt sich nicht völlig ausschliessen und ebenso lässt sich eine Bakterienbesiedelung nicht verhindern.

Das Konzept der GTR kann selbstverständlich nicht nur für Knochendefekte im Bereich der Zähne, sondern auch bei anderen am Körper auftretenden Knochendefekten angewandt werden.

Aufgabe der Erfindung ist die Auffindung eines Materials, mit welchem im Sinne der GTR auf einfache Weise eine Art Deckel über dem Knochendefekt erzeugt werden kann, wobei eine gute Bioverträglichkeit des Materials das längerzeitige Verbleiben in situ ermöglichen und ein ungestörtes Knochenwachstum gewährleisten soll, ohne dass es zu Bindegewebsbildung oder Bakterienbesiedelung kommt.

Erfindungsgemäss wurde festgestellt, dass von der Problemlösung durch das Prinzip einer semipermeablen Membran oder einem solchen Gewebe abgegangen werden muss und ein fester, dichter Wundverschluss zum Ziel führt, wobei sich Glasionomerzement in hervorragender Weise eignet, den Deckel über dem Knochendefekt aufzubauen.

Die Erfindung betrifft damit die Verwendung von Glasionomerzement zur Herstellung von Deckeln über Knochensubstanzdefekten und Knochenhohlräumen.

Durch die plastische Verformbarkeit des Glasionomerzements ist intraoperativ das Ausbilden des Deckels stark erleichtert, ausserdem vereinfacht die Haftung des Zements an der Knochen- und Zahnsubstanz die Stabilisierung des Deckels in situ. Durch die Stabilität des Deckels aus Glasionomerzement ist das Vernähen und die Stabilisierung des darüberliegenden Weichgewebes problemlos möglich. Aufgrund der guten Gewebsverträglichkeit kann der Deckel aus Glasionomerzement über mehrere Monate in situ verbleiben. Überraschenderweise hat sich nach der Entfernung nach ca. 3 Monaten ein neuer Zahnhalteapparat in physiologischer Weise gebildet, so dass die gelockerten Zähne befestigt werden und erhalten werden können. In vielen Fällen tritt praktisch eine restitutio ad integrum ein. Ein Einwachsen von Bindegewebe oder eine Bakterienbesiedelung konnte nicht beobachtet werden.

Der für die erfindungsgemässen Zwecke geeignete Glasionomerzement enthält vorzugsweise
(a) ein Aluminiumfluorosilikatglas,
(b) mindestens eine polymere Polysäure mit einem mittleren Molekulargewicht > 500,
(c) Wasser und
(d) gegebenenfalls ein chelatbildendes Mittel.

Als Bestandteil (a) können die in der DE-A-20 61 513 und der EP-A-0 023 013 beschriebenen Calciumaluminiumfluorosilikatgläser und die in der EP-A-0 241 277 beschriebenen Strontiumaluminiumfluorosilikatgläser eingesetzt werden. Die erfindungsgemäss verwendeten Aluminiumfluorosilikatglas-Pulver bestehen neben Sauerstoff vorzugsweise aus:

| Bestandteil | Berechnet als | Gew.-% |
|---|---|---|
| Si | SiO₂ | 20 - 60 |
| Al | Al₂O₃ | 10 - 50 |
| Ca | CaO | 0 - 40 |
| Sr | SrO | 0 - 40 |
| F | F | 1 - 40 |
| Na | Na₂O | 0 - 10 |
| P | P₂O₅ | 0 - 10 |

wobei mindestens 1 Gew.-% CaO und/oder SrO enthalten sein müssen und insgesamt 0 bis 20 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 bis 20 Gew.-% La₂O₃ können die Gläser röntgensichtbar gemacht werden.

Vorteilhaft bestehen die Pulverteilchen aus:

| | |
|---|---|
| Si als SiO₂ | 25 - 50 Gew.-% |
| Al als Al₂O₃ | 10 - 40 Gew.-% |
| Ca als CaO | 0 - 35 Gew.-% |
| Sr als SrO | 0 - 35 Gew.-% |
| F | 5 - 30 Gew.-% |
| Na als Na₂O | 0 - 8 Gew.-% |
| P als P₂O₅ | 1 - 10 Gew.-% |

wobei mindestens 10 Gew.-% Ca (berechnet als CaO) und/oder Sr (berechnet als SrO) enthalten sein müssen und 0 - 10 Gew.-% an B₂O₃, Bi₂O₃, ZnO, MgO, SnO₂, TiO₂, ZrO₂, La₂O₃ oder anderen Oxiden 3-wertiger Lanthanoide, K₂O, WO₃, GeO₂ sowie weiteren Zusätzen, welche die Eigenschaften nicht beeinträchtigen und physiologisch unbedenklich sind.

Besonders bevorzugt verwendete Pulver enthalten

| | |
|---|---|
| Si als SiO₂ | 25 - 45 Gew.-% |
| Al als Al₂O₃ | 20 - 40 Gew.-% |
| Ca als CaO | 10 - 30 Gew.-% |
| F | 10 - 30 Gew.-% |
| Na als Na₂O | 1 - 8 Gew.-% |
| P als P₂O₅ | 1 - 10 Gew.-% |

Die erfindungsgemäss verwendeten Glaspulver weisen eine durchschnittliche Korngrösse (Gewichtsmittel) von wenigstens 1 µm und bevorzugt mindestens 3 µm auf. Die durchschnittliche Korngrösse (Gewichtsmittel) beträgt 1 - 20 µm, bevorzugt 3 - 15 µm und besonders bevorzugt 3 - 10 µm. Die Teilchen haben eine maximale Korngrösse von 150 µm, vorzugsweise 100 µm, besonders 60 µm.

Die so erhaltenen Pulver werden dann gegebenenfalls einer Oberflächenbehandlung gemäss der europäischen Patentschrift 0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so dass sich praktisch keine löslichen Calcium- oder Strontiumsalze mehr auf der Oberfläche der Pulverteilchen befinden.

Die als Bestandteil (b) einzusetzenden polymeren Polysäuren können auch die bei der Herstellung von Glasionomerzementpulvern bekannten Polycarbonsäuren, z.B. Polymaleinsäure, Polyacrylsäure, Polyitaconsäure sowie Gemische davon oder Copolymere, insbesondere die aus der EP-A-0 024 056 bekannten Maleinsäure-Acrylsäure-Copolymeren und/oder Acrylsäure-Itaconsäure-Copolymeren sein. Das mittlere Molekulargewicht der erfindungsgemäss einzusetzenden Polycarbonsäuren beträgt mehr als 500. Vorteilhaft ist ein mittleres Molekulargewicht von 1.000 bis 20.000, besonders bevorzugt sind 3.000 bis 10.000. Die Polysäure wird vorzugsweise in Konzentrationen von 5 bis 50 Gew.-%, bezogen auf Bestandteil (a), eingesetzt.

Als polymere Polysäure geeignet sind auch Polyphosphonsäuren, z.B. Polyvinylphosphonsäure. Diese Polyphosphonsäuren können die oben genannten Polycarbonsäuren ganz oder teilweise ersetzen.

Der Bestandteil (c), das Wasser, wird in Mengen von 5 bis 70 Gew.-%, vorzugsweise 15 bis 40 Gew.-%, bezogen auf das Gesamtgewicht, eingesetzt.

Als Bestandteil (d) kann ein chelatbildendes Mittel, wie es in der DE-A-23 19 715 beschrieben ist, enthalten sein. Vorzugsweise wird als Chelatbildner Weinsäure eingesetzt. Die Chelatbildner können in Konzentrationen von 0,1 bis 10, vorzugsweise 3 bis 8 Gew.-%, bezogen auf die Gesamtmasse, eingesetzt werden.

Um hohe Lagerstabilität vor der Anwendung zu erhalten, empfiehlt sich ein Zusatz von Konservierungsstoffen, z.B. Benzoesäure, insbesondere zur trockenen Polysäure.

Zusätze zur Einstellung der Viskosität (z. B. pyrogene Kieselsäure) sind möglich. Geeignete Konzentrationen sind 0,1 bis 10, vorzugsweise 1 bis 5 Gew.-%, bezogen auf die Gesamtmasse.

Der Glasionomerzement kann darüber hinaus gemäss DE 40 19 617 Chemotherapeutika enthalten. Geeignete Chemotherapeutika sind beispielsweise Zytostatika, insbesondere Methotrexat, Vincristin, Cisplatin, Cyclophosphamid oder Antibiotika, insbesondere die Gyrasehemmer, wie beispielsweise Ciprofloxacin, Ofloxacin, Norfloxacin und deren Salze sowie Aminoglycosid-Antibiotika, insbesondere die Klasse der Lincomycine. Ganz besonders bevorzugt sind Clindamycin und Lincomycin sowie deren Salze und Derivate. Auch die Kombination mehrerer Wirkstoffe kann geeignet sein, um spezielle Indikationsgebiete optimal zu versorgen. Bezüglich geeigneter Gyrasehemmer wird auf W. Stille, FAC Band 6-10, 1987, S. 1575-1583 verwiesen. Alle dort beschriebenen Verbindungen kommen in Betracht.

Die Konzentration der Chemotherapeutika beträgt max. 10 Gew.-%, bevorzugt max. 3 Gew.-%, bezogen auf das Gesamtgewicht des Glasionomerzements und Chemotherapeutika. Besonders bevorzugt ist ein Bereich von 0,01 bis 3 Gew.-%.

Bevorzugt wird der Glasionomerzement in einer Kapsel angeboten, die Pulver und Flüssigkeit zunächst getrennt enthält. Nach Zusammenfügen von Pulver und Flüssigkeit wird die Masse durch mechanisches Anmischen mit einem üblichen Schüttelgerät zubereitet.

Vorzugsweise kommt eine Applikationskapsel nach EP-A-0 157 121 zur Anwendung.

Wichtig ist, dass die Ausgangskomponenten (Pulver und Flüssigkeit) steril bereitgestellt werden. Die Sterilisation kann dabei durch Hitzebehandlung, Sterilfiltration (Flüssigkeit) oder insbesondere durch Gammabestrahlung erfolgen.

Die Aufteilung der wesentlichen Bestandteile des Glasionomerzements
(a) Glaspulver
(b) polymere Polysäure
(c) Wasser
(d) Chelatbildner
kann auf verschiedene Weise erfolgen:

| Pulver | Flüssigkeit |
|---|---|
| (a) | (b) + (c) + (d) |
| (a) + (b) | (c) + (d) |
| (a) + (b) + (d) | (c) |

Möglich sind auch Paste/Paste-Aufteilungen, bei denen z.B. mit (c) kombiniert werden könnte.

Die erfindungsgemässe Verwendung des Glasionomerzements geschieht zur Heilung von Knochensubstanzdefekten sowie Knochenkontinuitätsunterbrechungen. Insbesondere Knochendefekte im Kieferbereich als Folge von Parodontalerkrankungen können durch die erfindungsgemässe Verwendung des Glasionomerzements regeneriert werden. Der parodontale Höhenabbau, der parodontale Seitenabbau, der parodontale Furkationsbefall sowie die Behandlung des atrophierten Alveolarfortsatzes in der dentalen Implantologie seien hierbei beispielshaft aufgezählt. Ebenso können zystische Knochendefekte im Skelettsystem durch Anbringen eines Glasionomerzementdeckels knöchern regeneriert werden.

### BEISPIEL

Zum Einsatz kommt ein herkömmlicher Glasionomerzement:
Pulver: Calciumaluminiumfluorosilikatglas, mittlere Teilchengrösse ca. 8 µm
Flüssigkeit: Lösung aus 39 g eines Copolymeren (1 : 1) aus Acrylsäure und Maleinsäure und 10 g Weinsäure in 51 g destilliertem Wasser.

340 mg der Flüssigkeit sind in ein Folienkissen gefüllt, welches an einer Applikationskapsel gemäss EP-B 0 157 121 angebracht ist. Die Hauptkammer der Applikationskapsel ist mit 860 mg des Zementpulvers gefüllt. Die Kapsel ist verblistert und mit Gammastrahlen sterilisiert.

Ein älterer Patient hat im Unterkiefer am ersten Molaren einen Furkationsbefall. Dies bedeutet, dass der Zahnhalteapparat an den Wurzelfurkationen atrophiert ist. Der Defekt ist in bucco-lingualer Richtung durch die Furkation durchgängig. Dies ist ein Furkationsbefall Klasse III. Es erfolgt ein parodontal-chirurgischer Eingriff. Die Schleimhaut wird vom Zahn und Alveolarfortsatz abgeklappt. Der Zahn wird gesäubert, curettiert und gescaled. Der Knochensubstanzverlust sowie der Verlust an Zahnhaltegewebe wird deutlich. Nun wird unter Verwendung des sterilen Glasionomerzements ein Deckel zur Therapie dieses Substanzverlustes in situ erstellt. Die Zementkapsel wird 10 Sekunden in einem üblichen Mischer angemischt. Der entnommene Zement wird in Form eines Zeltdaches vom Knochen an den Zahn geformt. Der Glasionomerzement verbindet sich sowohl mit dem Knochen als auch mit der Zahnsubstanz. Es erfolgt damit ein dichter Abschluss sowohl zum Zahn als auch zum Alveolarknochen. Das Einwachsen von Bindegewebe und Epithel wird durch den sehr dichten Verschluss verhindert. Diese Modellation geschieht sowohl auf der lingualen als auch auf der bukkalen Zahnfläche. Zwischen beiden "Knochendeckeln" verbleibt ein Hohlraum um die Wurzelfurkation. Dieser Hohlraum füllt sich intra operationem mit Blut. Aus dem Knochenmark sowie aus dem subepithelialen Bindegewebe der Gingiva orientieren sich Progenitorzellen, welche das parodontale Ligament an den ehemals freiliegenden Wurzelflächen neu in diesem Hohlraum bilden. Nach erfolgter Modellation und Überprüfung des Operationsergebnisses wird die Schleimhaut wieder über dem Defekt zugenäht. Der Deckel bleibt ca. 3 Monate in situ. Bei Beschwerdefreiheit kann der Deckel auch länger in situ verbleiben. Nach etwa 3 Monaten hat sich ein neuer Zahnhalteapparat an den Wurzelfurkationen gebildet. Der Zahn kann so erhalten werden. Ein Furkationsbefall führt mit den herkömmlichen parodontal-therapeutischen Möglichkeiten zur Extraktion des Zahnes. Mit Hilfe der Herstellung eines entsprechenden Knochendeckels aus Glasionomerzement lässt sich, wie hier gezeigt, der parodontale Höhen- und Seitenabbau sowie der Furkationsbefall therapieren.

## Patentansprüche

1. Verwendung von Glasionomerzement zur Herstellung von Deckeln über Knochensubstanzdefekten und Knochenhohlräumen.

2. Verwendung nach Anspruch 1, dadurch gekennzeichnet, daß der Glasionomerzement enthält
(a) ein Aluminiumfluorosilikatglas,
(b) mindestens eine polymere Polysäure mit einem mittleren Molekulargewicht >500,
(c) Wasser und
(d) gegebenenfalls ein chelatbildendes Mittel.

3. Verwendung nach den Ansprüchen 1 oder 2, dadurch gekennzeichnet, daß der Glasionomerzement in mindestens zwei räumlich voneinander getrennten Teilmassen vorliegt.

4. Verwendung nach Anspruch 3, dadurch gekennzeichnet, daß mindestens eine Teilmasse in fester, vorzugsweise pulverförmiger Form und mindestens eine andere Teilmasse in flüssiger oder pastöser Form vorliegen.

5. Verwendung nach Anspruch 4, dadurch gekennzeichnet, daß die feste Teilmasse den Bestandteil (a) und die flüssige Teilmasse die Bestandteile (b), (c) und gegebenenfalls (d) enthalten.

6. Verwendung nach Anspruch 4, dadurch gekennzeichnet, daß die feste Teilmasse die Bestandteile (a) und (b) und die flüssige Teilmasse die Bestandteile (c) und gegebenenfalls (d) enthalten.

7. Verwendung nach Anspruch 4, dadurch gekennzeichnet, daß die feste Teilmasse die Bestandteile (a), (b) und gegebenenfalls (d), und die flüssige Teilmasse den Bestandteil (c) enthalten.

8. Verwendung nach den Ansprüchen 1 bis 7, dadurch gekennzeichnet, daß der Glasionomerzement zusätzlich ein oder mehrere Chemotherapeutika enthält.

## Claims

1. Use of glass ionomer cement to produce coverings over bone substance defects and bone cavities.

2. Use according to claim 1, characterized in that the glass ionomer cement contains
(a) an aluminium fluorosilicate glass,
(b) at least one polymeric polyacid with an average molecular weight of 500,
(c) water and
(d) optionally a chelating agent.

3. Use according to claims 1 or 2, characterized in that the glass ionomer cement is present in at least two partial compositions which are spatially separated from each other.

4. Use according to claim 3, characterized in that at least one partial composition is present in solid, preferably powdery form and at least one other partial composition is present in liquid or paste form.

5. Use according to claim 4, characterized in that the solid partial composition contains constituent (a) and the liquid partial composition contains constituents (b), (c) and optionally (d).

6. Use according to claim 4, characterized in that the solid partial composition contains constituents (a) and (b) and the liquid partial composition contains constituents (c) and optionally (d).

7. Use according to claim 4, characterized in that the solid partial composition contains constituents (a), (b) and optionally (d) and the liquid partial composition contains constituent (c).

8. Use according to claims 1 to 7, characterized in that the glass ionomer cement additionally contains one or more chemotherapeutics.

## Revendications

1. Utilisation de ciment ionomère-verte pour la préparation de couvercles sur les défauts des substances osseuses et les évidements osseux.

2. Utilisation selon la revendication 1, caractérisée en ce que le ciment ionomère-verte contient
(a) un verte de fluorosilicate d'aluminium,
(b) au moins un polyacide polymère ayant un poids moléculaire moyen supérieur à 500,
(c) de l'eau et
(d) le cas échéant un agent chélatant.

3. Utilisation selon les revendications 1 ou 2, caractérisée en ce que le ciment ionomère-verte se présente en au moins deux masses partielles séparées l'une de l'autre dans l'espace.

4. Utilisation selon la revendication 3, caractérisée en ce qu'au moins une masse partielle se présente sous forme solide, de préférence pulvérulente, et en ce qu'au moins une autre masse partielle se présente sous forme liquide ou pâteuse.

5. Utilisation selon la revendication 4, caractérisée en ce que la masse partielle solide contient le composant (a) et en ce que la masse partielle liquide contient les composants (b), (c) et le cas échéant (d).

6. Utilisation selon la revendication 4, caractérisée en ce que la masse partielle solide contient les composants (a) et (b) et en ce que la masse partielle liquide contient les composants (c) et le cas échéant (d).

7. Utilisation selon la revendication 4, caractérisée en ce que la masse partielle solide contient les composants (a), (b) et le cas échéant (d), et en ce que la masse partielle liquide contient le composant (c).

8. Utilisation selon les revendications 1 à 7, caractérisée en ce que le ciment ionomère-verte contient en outre un ou plusieurs agents chimiothérapeutiques.
